# EUROPEAN PATENT SPECIFICATION

(11) **EP 4 269 376 B1**
(45) Date of publication and mention of the grant of the patent: **30.09.2026**
(21) Application number: 21910325.6
(22) Date of filing: 08.12.2021
(51) Int. Cl.: C09K 5/04, C07C 19/08, C07C 21/18, C23G 5/028, C09K 3/30, C11D 7/50, C08J 9/14, C07C 17/42

(54) **HYDROHALOOLEFIN COMPOSITION**
HYDROHALOGENOLEFINZUSAMMENSETZUNG
COMPOSITION D'HYDROHALO-OLÉFINE

(30) Priority: 22.12.2020 JP 2020212127
(43) Date of publication of application: 01.11.2023
(73) Proprietor: AGC INC., Chiyoda-ku Tokyo 100-8405 (JP)
(72) Inventor: CHINSOGA, Tamaki, Tokyo 100-8405 (JP); TANIGUCHI, Tomoaki, Tokyo 100-8405 (JP)
(74) Representative: Müller-Boré & Partner Patentanwälte PartG mbB
(86) International application number: PCT/JP2021/045200
(87) International publication number: WO 2022/138176

(56) References cited:
- WO-A1-2017/122801
- JP-A- 2016 079 095
- JP-A- 2016 519 053
- JP-A- 2018 150 502
- US-A1- 2018 148 394

## Description

### Technical Field

The present invention relates to a hydrohaloolefin composition.

### Background Art

A hydrohaloolefin such as a hydrofluoroolefin (HFO), a hydrochlorofluoroolefin (HCFO) or the like has a low global warming potential, and therefore has been expected to be substitutes for a chlorofluorocarbon in order to prevent global warming and ozone depletion.

The hydrohaloolefin may decompose over time or the like, and may not be sufficiently stable. Acids generated by decomposition thereof may cause corrosion of metals.

In Patent Document 1, an example is described in which the addition of a small amount of 1-chloro-2,2,3,3-tetrafluoropropane (HCFC-244ca) to 1-chloro-2,3,3-trifluoro-1-propene (HCFO-1233yd) inhibits decomposition during storage.

In Patent Document 2, an example is described in which the addition of a small amount of water to a composition containing 2,3,3,3-tetrafluoropropene (HFO-1234yf) or 1,3,3,3-tetrafluoropropene (HFO-1234ze) and oxygen inhibits decomposition during storage.

Patent Document 3 describes compositions based on F-1230za (1,1,3,3-tetrachloropropene), or a mixture consisting of F-1230za and F-1230zd (1,3,3,3-tetrachloropropene), the manufacture thereof, and also the use thereof especially for the production of F-1233zdE (trans-1-chloro-3,3,3-trifluoropropene), F-1234zeE (trans-1,3,3,3-tetrafluoropropene) and/or F-245fa (1,1,1,3,3-pentafluoropropane).

### Related Art Documents

### Patent Documents

Patent Document 1: Japanese Patent Application Laid-Open (JP-A) 2020-172657
Patent Document 2: Japanese Patent No. 5962827
Patent Document 3: US 2018/148394 A1

### SUMMARY OF INVENTION

### Technical Problem

Patent Documents 1 and 2 disclose HCFC-244ca or water as a compound that contributes to stability, but further diversification of technology has been required.

An object in the present disclosure is to provide a hydrohaloolefin composition with excellent stability.

### Solution to Problem

The present disclosure includes the following aspects.
[1] A hydrohaloolefin composition including a hydrohaloolefin as a main component, further including 1,1,2,3-tetrafluorobutane,
   in which a content of the 1,1,2,3-tetrafluorobutane is from 0.001 to 0.1% by mass with respect to a total amount of organic compounds.
[2] The hydrohaloolefin composition according to [1], further including 2,4,4,4-tetrafluoro-1-butene,
   in which a total content of the 1,1,2,3-tetrafluorobutane and the 2,4,4,4-tetrafluoro-1-butene is from more than 0.001% by mass to 0.4% by mass with respect to a total amount of organic compounds.
[3] The hydrohaloolefin composition according to [1] or [2], in which the main component is a hydrochlorofluoroolefin.
[4] The hydrohaloolefin composition according to [3], in which the main component is 1-chloro-2,3,3,3-tetrafluoropropene.
[5] The hydrohaloolefin composition according to any one of [1] to [4], which is at least one selected from the group consisting of a working fluid, a foaming agent, a diluent solvent, a cleaning agent, a propellant, an extinguishing agent, a gaseous dielectric, an etching gas, and a molten metal cover gas, as application.
[6] The hydrohaloolefin composition according to [5], which is in a form of an aerosol.
[7] The hydrohaloolefin composition according to [5], in which the working fluid is a working fluid for thermal cycling.
[8] The hydrohaloolefin composition according to [5], in which the cleaning agent is a cleaning agent for cleaning at least one selected from the group consisting of a carbon, an oil, and a dust adhering via carbon or oil.
[9] The hydrohaloolefin composition according to [5], in which the cleaning agent is a cleaning agent for cleaning an article made of a metal, a resin, a rubber, a glass, a ceramic, a natural fiber, a synthetic fiber, or a composite material including two or more thereof.
[10] The hydrohaloolefin composition according to [5], in which the propellant includes at least one selected from the group consisting of a pressor agent and an atomizing agent.
[11] The hydrohaloolefin composition according to [5], in which the gaseous dielectric includes at least one selected from the group consisting of a liquefied gas and a compressed gas.

### Advantageous Effects of Invention

According to the present disclosure, a hydrohaloolefin composition with excellent stability can be provided.

### DESCRIPTION OF EMBODIMENTS

The definitions of the terms below apply in the specification and claims.
"Hydrohaloolefin" is a general term for unsaturated hydrocarbon compounds including a hydrogen atom and at least one selected from the group consisting of a fluorine atom, a chlorine atom, a bromine atom and an iodine atom.
"Organic compound" is a general term for compounds including a carbon atom, excluding an oxide of carbon and a carbonate of metal.
"Main component of composition" means a component having the largest content with respect to a total mass of a composition.

An abbreviation or alias of a compound may be written in parentheses after a compound name. The abbreviation or alias may be used instead of compound name.

An unsaturated hydrocarbon compound have geometric isomers of a Z-isomer (cis-isomer) and an E-isomer (trans-isomer) depending on a position of a substituent attached to carbons having a double bond. In a case in which a compound name or an abbreviation of a compound is used without particular mention for a compound in which Z-isomer and E-isomer may exist, it means that it is a Z-isomer, an E-isomer, or a mixture of Z-isomer and E-isomer in any ratio. In a case in which (Z) or (E) is attached to an end of a compound name or an abbreviation, it indicates that the compound is in Z-form or E-form.

The numerical range indicated using an expression "to" means a numerical range including numerical values described before and after "to" as a minimum value and a maximum value, respectively.

In a numerical range described in stepwise, the upper limit value or the lower limit value described in one numerical range may be replaced with the upper limit value or the lower limit value in another numerical range described in stepwise. Further, in a numerical range, the upper limit value or the lower limit value described in the numerical range may be replaced with a value shown in Examples.

### "(Meth)acryl" means acryl or methacryl.

<Hydrohaloolefin Composition>

A hydrohaloolefin composition in the present disclosure (hereinafter also referred to as the present composition) contains two or more organic compounds, and the main component is a hydrohaloolefin.

A content of the main component with respect to the total mass of the composition is preferably 50% by mass or more, more preferably 70% by mass or more, still more preferably 80% by mass or more, particularly preferably 90% by mass or more, and extremely preferably 99% by mass or more.

### [Hydrohaloolefin]

Examples of the hydrohaloolefin include a hydrofluoroolefin (HFO), a hydrochlorofluoroolefin (HCFO), and a hydrochloroolefin (HCO). The hydrohaloolefin may be used singly or in combination of two or more.

Specific examples of the hydrofluoroolefin (HFO) as a main component include 1,1,2-trifluoroethylene (HFO-1123), 1-fluoropropene (HFO-1261ze), 1,2-difluoropropene (HFO-1252ye), 1,3,3,3-tetrafluoropropene (HFO-1234ze), 1,1,2,3-tetrafluoropropene (HFO-1234yc), 2,3,3,3-tetrafluoropropene (HFO -1234yf), 1,2,3,3-tetrafluoropropene (HFO-1234ye), 1,1,3,3-tetrafluoropropene (HFO-1234zc), 1,1,2,3,3-pentafluoropropene (HFO-1225yc), 1,1,3,3,3-pentafluoropropene (HFO-1225zc), 1,1,2-trifluoropropene (HFO-1243yc), 1,2,3-trifluoropropene (HFO-1243ye), 3,3,3-trifluoropropene (HFO-1243zf), 1,3,3-trifluoropropene (HFO-1243ze), 3,3-difluoropropene (HFO-1252zf), 2-fluoropropene (HFO-1261yf), 1,1,1,4,4,4-hexafluoro-2-butene (HFO-1336mzz), 2,3,3,4,4,4-hexafluoro-1-butene (HFO-1336mcyf), 1,3,3,4,4,4-hexafluoro-1-butene (HFO-1336ze), 2,4,4,4-tetrafluoro-1-butene (HFO-1354yf), 1,1,1,2,4,4,5,5,5-nonafluoropentene (HFO-1429myz), 1,1,1,4,4,5,5,5-octafluoropenta-2-ene (HFO-1438mzz), 1,3,4,4,4-pentafluoro-3-trifluoromethyl-1-butene (HFO-1438ezy), (C₂F₅)(CF₃)C=CH₂, (CF₃)₂CFCH=CF₂, (CF₃)₂CFCF=CHF, 1,1-difluoroethylene (HFO-1132a), 1,2-difluoroethylene (HFO-1132), and 1,2,3,3,3-pentafluoro-1-propene ( HFO-1225ye).

Specific examples of the hydrochlorofluoroolefin (HCFO) as main components include 1-chloro-2,3,3,3-tetrafluoropropene (HCFO-1224yd), 1-chloro-2,3,3-trifluoropropene (HCFO-1233yd), 2-chloro-3,3,3-trifluoropropene (HCFO-1233xf), 2-chloro-1,1,3-trifluoropropene (HCFO-1233xc), 2-chloro-1,3,3-trifluoropropene (HCFO-1233xe), 1-chloro-1,2,3-trifluoropropene (HCFO-1233yb), 1-chloro-1,3,3-trifluoropropene (HCFO-1233zb), 3-chloro-1,1,3-trifluoropropene (HCFO-1233zc), 3-chloro-1,3,3-trifluoropropene (HCFO-1233ze), 1-chloro-3,3,3-trifluoropropene (HCFO-1233zd), 2,3,3-trichloro-3-fluoropropene (HCFO-123 1xf), 2,3-dichloro-3,3-difluoropropene (HCFO-1232xf), 1,2-dichloro -3,3,3-trifluoropropene (HCFO-1223xd), 2-chloro-1,1,3,3-tetrafluoropropene (HCFO-1224xc), 2-chloro-1,3,3,3-tetrafluoropropene (HCFO-1224xe), 1,1-dichloro-2-fluoroethylene (HCFO-1121a), 1,2-dichloro-1-fluoroethylene (HCFO-1121), 1-chloro-1-fluoroethylene (HCFO - 1131a), 1-chloro-2-fluoroethylene (HCFO-1131), and 1-chloro-2,2-difluoroethylene (HCFO-1122).

Specific examples of the hydrochloroolefin (HCO) as the main component include chloroethylene and 1,2-dichloroethylene.

The main component of the present composition is preferably hydrochlorofluoroolefin (HCFO), and more preferably HCFO-1224yd.

HCFO-1224yd may be HCFO-1224yd (Z), HCFO-1224yd (E), or a mixture thereof. The Z/E ratio, which represents a mass ratio of the Z isomer content to the E isomer content in HCFO-1224yd, may be from 100/0 to 0/100, preferably from 99.9/0.1 to 50/50, more preferably from 99.9/0.1 to 70/30, and still more preferably 99.9/0.1 to 99.0/1.0.

The present composition contains 1,1,2,3-tetrafluorobutane (HFC-374pee) in addition to the hydrohaloolefin as the main component.

A content of HFC-374pee is from 0.001 to 0.1% by mass, preferably 0.001 to 0.05% by mass, and more preferably 0.001 to 0.01% by mass, with respect to the total mass of the organic compounds in the present composition. Within the above range, a stability of the present composition is excellent.

The hydrohaloolefin may include 2,4,4,4-tetrafluoro-1-butene (HFO-1354yf). In a case in which HFO-1354yf is included, a total content of HFC-374pee and HFO-1354yf is preferably from more than 0.001 to 0.4% by mass, more preferably from 0.001 to 0.2% by mass, and still more preferably from 0.01 to 0.1% by mass, with respect to the total mass of organic compounds in the present composition. Within the above range, a stability of the present composition is excellent.

In a case in which the present composition contains HFO-1354yf, the main component is hydrohaloolefin other than HFO-1354yf.

The present composition may contain one or more other organic compound(s) other than the hydrohaloolefin as the main component (which may contain HFO-1354yf) and HFC-374pee.

Other organic compound(s) include a chlorofluoroolefin (CFO), a fluoroolefin, a chloroolefin, an olefin, a hydrofluorocarbon (HFC) other than HFC-374pee, a hydrochlorocarbon (HCC), a hydrochlorofluorocarbon (HCFC), a chlorofluorocarbon (CFC), a chlorocarbon, a fluorocarbon (FC), a hydrocarbon (HC), a hydrofluoroether (HFE), a chlorofluoroalkyne, an alcohol, an ether, a ketone, an organic acid, and a saturated hydrocarbon.

Furthermore, a halogenated methane including at least one selected from the group consisting of a bromine atom and an iodine atom may be included as necessary.

Specific examples of the chlorofluoroolefin (CFO) include 1,1-dichloro-2,3,3,3-tetrafluoropropene (CFO-1214ya), 1,1,2-trichloro-2-fluoroethylene, 1,1,2-trifluoro-2-chloroethylene (HCFO-1113) and 2-chloro-1,1,3,3,3-pentafluoro-1-propene (CFO-1215xc).

Specific examples of the fluoroolefin include hexafluoropropene (FO-1216) and octafluoro-2-butene (FO-1318my).

Specific examples of the chloroolefin include tetrachloroethylene.

Specific examples of the olefin include ethylene and propylene.

Specific examples of the hydrofluorocarbon (HFC) other than HFC-374pee include 1,1,1,3,3-pentafluoropropane (HFC-245fa), 1,1,1,2,2-pentafluoropropane (HFC -245cb), 1,1,2,2,3-pentafluoropropane (HFC-245ca), 1,1,1,2,3-pentafluoropropane (HFC-245eb), 1,1,1,2, 3,3,3-heptafluoropropane (HFC-227ea), 1,1,1,2-tetrafluoropropane (HFC-254eb), 1,1,1,3-tetrafluoropropane (HFC-254fb), 1,1,1-trifluoropropane (HFC-263fb), 1,1,1,2,3,3-hexafluoropropane (HFC-236ea), 1,1,1,3,3,3-hexafluoropropane (HFC-236fa), 2-fluoropropane (HFC-281ea), 2-chloro-1,1,1,2-tetrafluoropropane (HCFC-244bb), 1,1,2,2-tetrafluoroethane (HFC-134), 1,1,1,2-tetrafluoroethane (HFC-134a), 1,2-difluoroethane (HFC-152), 1,1-difluoroethane (HFC-152a), difluoromethane (HFC-32), 1,1,1,2,2-pentafluoroethane (HFC-125), 1,1,2-trifluoroethane (HFC-143), 1,1,1-trifluoroethane (HFC-143a), 1,1,1,3,3-pentafluorobutane (HFC-365mfc), fluoroethane (HFC-161), 1,1,1,2,2,3,4,5,5,5-decafluoropentane (HFC-43-10mee), trifluoromethane (HFC-23), and fluoromethane (HFC-41).

Specific examples of the hydrochlorocarbon (HCC) include chloroform, 1,1,1,2,3-pentachloropropane (HCC-240db), and 2-chloropropane.

Specific examples of the hydrochlorofluorocarbon (HCFC) include chlorodifluoromethane (HCFC-22), chlorofluoromethane (HCFC-31), trichlorodifluoroethane (HCFC-122), 1,1,2-trichloro-1,2-difluoroethane (HCFC-122a), 1,1,1-trichloro-2,2-difluoroethane (HCFC-122b), 2,2-dichloro-1,1,1-trifluoroethane (HCFC-123), 2-chloro-1,1,1,2-tetrafluoroethane (HCFC-124), 1-chloro-1,1,2,2-tetrafluoroethane (HCFC-124a), 2-chloro-1,1,1-trifluoroethane (HCFC-133a), 1,1-dichloro-1-fluoroethane (HCFC-141b), 1,1-difluoro-2-chloroethane (HCFC-142), 1-chloro-1,2-difluoroethane (HCFC-142a), 1-chloro-1,1-difluoroethane (HCFC-142b), 3,3-dichloro-1,1,1,2,2-pentafluoropropane (HCFC-225ca), 1,3-dichloro-1,1,2,2,3-pentafluoropropane (HCFC-225cb), 2,2-dichloro-1,1,1-trifluoropropane (HCFC-243ab), 2,3-dichloro-1,1,1-trifluoropropane (HCFC-243db), 2-chloro-1,1,1,2-tetrafluoropropane (HCFC-244bb), 3-chloro-1,1,1,2,2,3-hexafluoropropane (HCFC-226ca), 1-chloro-1,1,2,3,3,3-hexafluoropropane (HCFC-226ea), and 2-chloro-1,1,1,3,3-pentafluoropropane (HCFC-235da).

Specific examples of the chlorofluorocarbon (CFC) include trichlorofluoromethane (CFC-11), dichlorodifluoromethane (CFC-12), chlorotrifluoromethane (CFC-13), trichlorotrifluoroethane (CFC-113), 1,1,1-trichloro-2,2,2-trifluoroethane (CFC-113a), 1,2-dichloro-1,1,2,2-tetrafluoroethane (CFC-114), 1,1-dichloro-1,2,2,2-tetrafluoroethane (CFC-114a), chloropentafluoroethane (CFC-115), dichlorohexafluoropropane (CFC-216), 2,2,3,3-tetrachlorohexafluorobutane (CFC-316), and dichlorooctafluorobutane (CFC-318).

Specific examples of the chlorocarbon include dichloromethane (methylene chloride) and trichloroethane.

Specific examples of the fluorocarbon (FC) include 1,1,1,2,2,2-hexafluoroethane (FC-116), octafluoropropane (FC-218), and 1,1,1,2,2,3,3-heptafluoropropane (FC-227ca).

Specific examples of the hydrocarbon (HC) include methane, ethane, propane, n-butane, isobutane, n-pentane, isopentane, neopentane, cyclopentane, n-hexane, isohexane, and heptane.

Specific examples of the hydrofluoroethers (HFE) include CHF₂-O-CHF₂, CHF₂-O-CH₂F, CH₂F-O-CH₂F, CH₂F-O-CH₃, cyclo-CF₂-CH₂-CF₂-O, cyclo-CF₂-CF₂-CH₂-O, CHF₂-O-CF₂-CHF₂, CF₃-CF₂-O-CH₂F, CHF₂-O-CHF-CF₃, CHF₂-O-CF₂-CHF₂, CH₂F-O-CF₂-CHF₂, CF₃-O-CF₂-CH₃, CHF₂-CHF-O-CHF₂, CF₃-O-CHF-CH₂F, CF₃-CHF-O-CH₂F, CF₃-O-CH₂-CHF₂, CHF₂-O-CH₂-CF₃, CHF₂-CF₂-O-CH₂F, CHF₂-O-CF₂-CH₃, CHF₂-CF₂-O-CH₃, CH₂F-O-CHF-CH₂F, CHF₂-CHF-O-CH₂F, CF₃-O-CHF-CH₃, CF₃-CHF-O-CH₃, CHF₂-O-CH₂-CHF₂, CF₃-O-CH₂-CH₂F, CF₃-CH₂-O-CH₂F, CF₂H-CF₂-CF₂-O-CH₃, CF₃CF₂CF₂-O-CH₃, and C₄H₉-O-CH₃.

Specific examples of the chlorofluoroalkynes include 1-chloro-3,3,3-trifluoro-1-propyne (CF₃-C≡CCl).

Specific examples of the alcohol include methanol, ethanol, propanol, and isopropanol.

Specific examples of the ether include dimethyl ether, methyl ethyl ether, diethyl ether, methyl propyl ether, methyl isopropyl ether, ethyl propyl ether, ethyl isopropyl ether, dipropyl ether, diisopropyl ether, dimethoxymethane, diethoxyethane, dipropoxymethane, and dibutoxymethane.

Specific examples of the ketone include ketone, methyl ethyl ketone, methyl isobutyl ketone, and perfluoroethyl isopropyl ketone.

Specific examples of the organic acid include methyl formate, ethyl formate, and formic acid.

Specific examples of the saturated hydrocarbon include methane, ethane, propane, butane, isobutane, pentane, isopentane, cyclopentane, and hexane.

Specific examples of the halogenated methane containing at least one selected from the group consisting of bromine atom and iodine atom include (mono)iodomethane (CH₃I), diiodomethane (CH₂I₂), dibromomethane (CH₂Br₂), bromomethane (CH₃Br), dichloromethane (CH₂Cl₂), chloroiodomethane (CH₂ClI), dibromochloromethane (CHBr₂Cl), methane tetraiodide (CI₄), carbon tetrabromide (CBr₄), bromotrichloromethane (CBrCl₃), dibromodichloromethane (CBr₂Cl₂), tribromofluoromethane (CBr₃F), fluorodiiodomethane (CHFI₂), difluorodiiodomethane (CF₂I₂), dibromodifluoromethane (CBr₂F₂), and trifluoroiodomethane (CF₃I).

A content of other organic compound(s) is preferably 1.5% by mass or less, more preferably 1.0% by mass or less, and still more preferably 0.5% by mass or less, with respect to the total mass of the organic compounds in the present composition..

The present composition may contain an inert gas in addition to the organic compound. Specific examples of the inert gas include carbon dioxide, nitrogen, oxygen, air (nitrogen: about 80% by volume, oxygen: about 20% by volume), argon, and helium.

A content of the inert gas may be 20% by volume or less, preferably 15% by volume or less, more preferably 10% by volume or less, still more preferably 5% by volume or less, and particularly preferably 1.5% by volume or less, with respect to a total volume of the gas phase portion when the present composition is filled in a closed container.

The present composition may contain water in addition to the organic compound. A content of water may be 200 ppm by mass or less, preferably 150 ppm by mass or less, more preferably 100 ppm by mass, and still more preferably 20 ppm by mass, with respect to the total amount of organic compounds in the present composition.

### <Manufacturing method>

The hydrohaloolefin contained in the present composition can be produced by a known production method.

For example, HCFO-1224yd may be obtained as an intermediate in a process of obtaining HFO-1234yf by reducing CFO-1214ya with hydrogen in the presence of a catalyst (hydrogen reduction reaction).

CFO-1214ya as a raw material may be produced using HCFC-225ca as a raw material, for example, by subjecting it to a dehydrofluorination reaction with an alkaline aqueous solution such as an aqueous potassium hydroxide solution in the presence of a phase transfer catalyst such as tetrabutylammonium bromide; or subjecting it to a dehydrofluorination reaction by a gas phase reaction in the presence of a catalyst such as chromium, iron, copper, or activated carbon.

HCFO-1224yd may be separated from an unreacted CFO-1214ya and HFO-1234yf by distilling the reaction solution obtained by the hydrogen reduction reaction using a normal distillation method or an extractive distillation method described in WO 2017/146190, thereby obtaining a purified composition containing HCFO-1224yd at high concentration. The purified composition may contain by-products from the hydrogen reduction reaction.

The HFC-374pee contained in the present composition may be contained in a purified composition obtained by producing a hydrohaloolefin and purifying thereof, may be added arbitrarily, or may be a combination thereof.

HFO-1354yf that may be contained in the present composition may be contained in the purified hydrohaloolefin composition, may be added arbitrarily, or may be a combination thereof.

A kind and a content of organic compounds contained in the present composition may be analyzed and identified using a GC-MS system.

As shown in Examples below, by adding HFC-374pee to a composition containing a hydrohaloolefin as a main component, decomposition of the hydrohaloolefin is suppressed and stability is improved. Although the reason is not clear, it is considered as follows.

HFC-374pee has a HFC properties, and is presumed to also have the properties of scavenging radicals, although it is not necessarily clear. Due to this action, it is thought that it exhibits a function as a stabilizer that suppresses decomposition of hydrohaloolefin such as HCFO-1224yd and stabilizes them.

### <Use>

The present composition has excellent stability and may be used for known uses for hydrohaloolefins. In addition, the present composition hardly corrodes metals even when it comes into contact with them, and may be applied to uses in which it comes into contact with metals.

Examples of uses for the present composition include a working fluid, a foaming agent, a diluent solvent, a cleaning agent, a propellant, an extinguishing agent, a gaseous dielectric, an etching gas, and a molten metal cover gas. Use of the present composition may be one type, or two or more types. The present composition may be used in a form of an aerosol or the like.

The working fluid using the present composition includes a working fluid for thermal cycling such as a refrigerant for refrigerator, a refrigerant for air conditioner, a working fluid for power generation system (waste heat recovery power generation, or the like), a working fluid for latent heat transport device (heat pipes, or the like), secondary cooling media, and the like.

Examples of the foaming agent using the present composition include a physical foaming agent used in a production of a thermoplastic resin foam and a thermosetting resin foam.

Specific examples of a thermoplastic resin contained in the thermoplastic resin foam include a polycarbonate resin, a polystyrene resin, a polyphenylene ether resin, an acrylonitrile-butadiene-styrene resin, a polyolefin resin, a polyvinyl chloride resin, a (meth)acryl resin, a polyester resin, a modified polyphenylene ether resin, a polyacetal resin, a polyetherimide resin, a polyethersulfone resin, a polyamide resin, a polysulfone resin, a polyetheretherketone resin, and a polyetherketone resin.

Specific examples of the thermosetting resin foam include a polyurethane foam, a polyisocyanurate foam, and a phenolic resin foam.

Specific examples of the polyurethane foam include a rigid urethane foam, a flexible urethane foam, a semi-rigid urethane foam, and an integral skin foam.

Specific examples of the diluent solvent using the present composition include: a solvent of a coating film-forming composition containing a functional ingredient such as a lubricant (a silicone lubricant, a fluorine lubricant or the like), a rust-preventive agent (a mineral oil, a synthetic oil or the like), a moisture-proof coating agent for water repellent treatment, an antifouling coating agent for antifouling treatment (a fingerprint removal and an adhesion prevention agent) or the like; a solvent for diluting a functional ingredient such as an insecticide, an insect repellent, a preservative or the like; a diluent solvent for chemical processes (an extraction solvent, a polymerization solvent), and the like.

Specific examples of the cleaning agent using the present composition include a cleaning agent for removing hydrophobic substances such as a degreasing cleaning agent, a flux cleaning agent, a precision cleaning agent, a dry cleaning agent, a pipe cleaning agent, a glass cleaners, a heat exchanger cleaner for refrigeration and air conditioning equipment, an oil stain cleaning agent; a cleaning agent for stain removal cleaning of clothes, or the like. The cleaning agent may be used in the form of an aerosol and the like.

In a method of cleaning with a cleaning agent using the present composition, the stain to be cleaned off includes a carbon, an oil, and a dust adhering via carbon or oil, which are attached to various objects to be cleaned. A fat and oil include a flux, a processing oil, a release agent, a sebum, a food oil, and a cosmetic. Examples of the processing oil include a cutting oil, a quenching oil, a rolling oil, a lubricating oil, a machine oil, a press processing oil, a punching oil, a drawing oil, an assembly oil, a wire drawing oil, a brake fluid, and the like. Examples of the cosmetic include a nail polish, a lipstick, and the like. Two or more of the above-described stain may adhere to various objects to be cleaned. The solvent composition in the present disclosure is preferably used for cleaning stains made of these oils, since it has excellent solubility in these oils compared to a conventional solvent composition such as HFC and HFE.

Examples of a material of an article to which the cleaning agent using the present composition may be applied include a metal, a resin, a rubber, a glass, a ceramic, a natural fiber, and a synthetic fiber. The article may also be an article made of a composite material containing two or more of these materials. Examples of the composite material include laminates of metal and resin. In particular, the solvent composition in the present disclosure may also be used for an article containing rubber material such as SBR or a resin material, which are affected by HCFO-1224yd.

The propellant using the present composition may be mixed with at least one selected from the group consisting of a pressor agent and an atomizing agent as a functional ingredient. Specifically examples of the pressor agent include compressed gas, and specifically examples of the atomizing agent include an insecticide, a deodorant, a lubricant, a rust-preventive agent, an antistatic agent, an extreme pressure agent, an antifog agent, a penetrant, a wetting agent, a paint, a cleansing agents, a cosmetic, a topical medication, a dye, an antifouling agent, a water repellent, and an oil repellent. The propellant may be used in the form of an aerosol or the like.

The gaseous dielectric using the present composition may be mixed with at least one selected from the group consisting of a liquefied gas and a compressed gas, and may be used in an electrical device. Specific examples of the electrical device include a circuit breaker, a current interrupter, a gas-insulated transmission line, a gas-insulated transformer, a gas-insulated substation, a gas-insulated switch, a gas-insulated disconnector, and a gas-insulated load switch.

Examples of usage of the etching gas using the present composition include a process of forming a circuit pattern of a semiconductor integrated circuit.

Examples of usage of the molten metal cover gas using the present composition include a gas (protective atmosphere) that covers a molten metal in order to prevent oxidation and evaporation of the molten metal in the crucible.

### EXAMPLE

Hereinafter, the present embodiments will be described in more detail using the following examples, but the present embodiments are not limited to these examples.

### <Evaluation Method>

### [Stability Test / Metal Corrosion Resistance Test]

A total of three metal pieces (20 mm×30 mm in length on one side and 2 mm in thickness) are prepared, in whice the metal pieces are made of SS400, Cu, and Al, respectively.

Three pieces of the metal pieces are placed in a pressure-resistant container made of stainless steel with a capacity of 200 mL and the container is sealed. The container is evacuated and charged with 60 g of the hydrohaloolefin composition to be tested.

Then, after storing the sealed pressure-resistant container in a constant temperature bath at 175 °C for 14 days, it is allowed to stand until it reaches room temperature (25 °C).

Each of the four absorption bottles is poured with 100 mL of pure water, and connected in series with pipes. The pressure container having room temperature (25 °C) is connected to the absorption bottle connected, a valve of the pressure-resistant container is gradually opened, the gas in the pressure-resistant container is introduced into water in the absorption bottles, and an acid component contained in the gas is extracted.

After extraction, the water in the first and second absorption bottles in order from the side closest to the pressure-resistant container was mixed, one drop of indicator (BTB: bromothymol blue) was added thereto, and titrated with a 1/100N NaOH alkaline standard solution to obtain a measured value. Also, the water in the third and fourth absorption bottles are mixed and titrated in the same manner to obtain a value of measurement blank. From the measured value and the value of measurement blank, an acid content contained in the hydrohaloolefin composition to be tested is determined as an HCl-equivalent concentration.

Based on the obtained acid content (HCl-equivalent concentration), the stability is evaluated according to the following criteria.
S (excellent): Acid content is less than 1 ppm by mass.
A (Good): Acid content is 1 ppm by mass or more and less than 5 ppm by mass.
B (Slightly Poor): Acid content is 5 ppm by mass or more.

After measuring the acid content, the three metal pieces is removed from the pressure-resistant container, and the appearance of the pieces are visually observed. The appearance is compared with each unused metal piece, and metal corrosion resistance is evaluated according to the following criteria.
S (excellent): All three metal pieces have the same luster as unused pieces, and none have rust.
A (Good): One or more metal piece(s) have lost their luster, but none have rust.
B (Slightly Poor): One or more piece(s) have slightly rusted at a surface, but none have rusted at whole surface.

Example 1 is a comparative example, and Examples 2 to 5 are examples.

### <Examples 1 to 5>

HCFC-225ca as a raw material was mixed with an aqueous metal hydroxide solution in the presence of a phase transfer catalyst, and subjected to a dehydrofluorination reaction to obtain CFO-1214ya. The obtained CFO-1214ya as a raw material was contacted with a catalyst, in which palladium is supported on activated carbon, in the gas phase of hydrogen and nitrogen, and subjected to a hydrogen reduction reaction to obtain a reaction liquid containing HFO-1234yf and HCFO-1224yd. The resulting reaction liquid was distilled and purified to obtain a purified composition containing HCFO-1224yd at a high concentration. Hydrohaloolefin composition of Example 1 was the resulting purified composition. Hydrohaloolefin compositions of Examples 2 to 5 were compositions in which HFC-374pee was added to the resulting purified compositions as shown in Table 1.

The compositions of the resulting hydrohaloolefin compositions were analyzed using a GC-MS system. Contents (unit: % by mass) of HCFO-1224yd(Z), HCFO-1224yd(E), HFC-374pee, HFO-1354yf, and other organic compounds with respect to a total mass of organic compounds in the hydrohaloolefin compositions were shown in Table 1. In Table 1, "<0.001" means below the detection limit.

The obtained hydrohaloolefin compositions were used as a test subject, and the stability and metal corrosion resistance were evaluated by the above method. The results were shown in Table 1.

**[Table 1]**

| | | Example 1 | Example 2 | Example 3 | Example 4 | Example 5 |
|---|---|---|---|---|---|---|
| Content with respect to total amount of organic compounds (% by mass) | HCFO-1224yd(Z) | 99.2 | 99.9 | 99.7 | 99.7 | 99.8 |
| | HCFO-1224yd(E) | 0.101 | 0.002 | <0.001 | <0.001 | <0.001 |
| | HFC-374pee | - | 0.001 | 0.006 | 0.007 | 0.010 |
| | HFO-1354yf | 0.097 | 0.070 | 0.029 | 0.051 | 0.013 |
| | Other organic compounds | 0.592 | 0.046 | 0.220 | 0.223 | 0.147 |
| Evaluation | Stability | B | A | S | S | S |
| | Metal corrosion resistance | B | A | S | S | S |

## Claims

1. A hydrohaloolefin composition comprising a hydrohaloolefin as a main component,
further comprising 1,1,2,3-tetrafluorobutane,
wherein a content of the 1,1,2,3-tetrafluorobutane is from 0.001 to 0.1% by mass with respect to a total amount of organic compounds.

2. The hydrohaloolefin composition according to claim 1, further comprising 2,4,4,4-tetrafluoro-1-butene,
wherein a total content of the 1,1,2,3-tetrafluorobutane and the 2,4,4,4-tetrafluoro-1-butene is from more than 0.001% by mass to 0.4% by mass with respect to a total amount of organic compounds.

3. The hydrohaloolefin composition according to claim 1 or 2, wherein the main component is a hydrochlorofluoroolefin.

4. The hydrohaloolefin composition according to claim 3, wherein the main component is 1-chloro-2,3,3,3-tetrafluoropropene.

5. The hydrohaloolefin composition according to any one of claims 1 to 4, which is at least one selected from the group consisting of a working fluid, a foaming agent, a diluent solvent, a cleaning agent, a propellant, an extinguishing agent, a gaseous dielectric, an etching gas, and a molten metal cover gas, as application.

6. The hydrohaloolefin composition according to claim 5, which is in a form of an aerosol.

7. The hydrohaloolefin composition according to claim 5, wherein the working fluid is a working fluid for thermal cycling.

8. The hydrohaloolefin composition according to claim 5, wherein the cleaning agent is a cleaning agent for cleaning at least one selected from the group consisting of a carbon, an oil, and a dust adhering via carbon or oil.

9. The hydrohaloolefin composition according to claim 5, wherein the cleaning agent is a cleaning agent for cleaning an article made of a metal, a resin, a rubber, a glass, a ceramic, a natural fiber, a synthetic fiber, or a composite material comprising two or more thereof.

10. The hydrohaloolefin composition according to claim 5, wherein the propellant comprises at least one selected from the group consisting of a pressor agent and an atomizing agent.

11. The hydrohaloolefin composition according to claim 5, wherein the gaseous dielectric comprises at least one selected from the group consisting of a liquefied gas and a compressed gas.

## Patentansprüche

1. Hydrohalogenolefinzusammensetzung, umfassend ein Hydrohalogenolefin als einen Hauptbestandteil,
weiter umfassend 1,1,2,3-Tetrafluorbutan,
wobei ein Gehalt des 1,1,2,3-Tetrafluorbutans von 0,001 bis 0,1 Massen-% bezogen auf eine Gesamtmenge organischer Verbindungen beträgt.

2. Hydrohalogenolefinzusammensetzung nach Anspruch 1, weiter umfassend 2,4,4,4-Tetrafluor-1-buten,
wobei ein Gesamtgehalt des 1,1,2,3-Tetrafluorbutans und des 2,4,4,4-Tetrafluor-1-butens im Bereich von mehr als 0,001 Massen-% bis 0,4 Massen-% bezogen auf eine Gesamtmenge organischer Verbindungen beträgt.

3. Hydrohalogenolefinzusammensetzung nach Anspruch 1 oder 2, wobei der Hauptbestandteil ein Hydrochlorfluorolefin ist.

4. Hydrohalogenolefinzusammensetzung nach Anspruch 3, wobei der Hauptbestandteil 1-Chlor-2,3,3,3-tetrafluorpropen ist.

5. Hydrohalogenolefinzusammensetzung nach einem der Ansprüche 1 bis 4, die als Anwendung mindestens eines, ausgewählt aus der Gruppe, bestehend aus einem Arbeitsfluid, einem Schäumungsmittel, einem Verdünnungslösungsmittel, einem Reinigungsmittel, einem Treibmittel, einem Löschmittel, einem gasförmigen Dielektrikum, einem Ätzgas und einem Deckgas für geschmolzenes Metall, aufweist.

6. Hydrohalogenolefinzusammensetzung nach Anspruch 5, die in einer Form eines Aerosols vorliegt.

7. Hydrohalogenolefinzusammensetzung nach Anspruch 5, wobei das Arbeitsfluid ein Arbeitsfluid für einen thermischen Kreislauf ist.

8. Hydrohalogenolefinzusammensetzung nach Anspruch 5, wobei das Reinigungsmittel ein Reinigungsmittel zum Reinigen mindestens eines, ausgewählt aus der Gruppe, bestehend aus einem Kohlenstoff, einem Öl und einem Staub, der über Kohlenstoff oder Öl anhaftet, ist.

9. Hydrohalogenolefinzusammensetzung nach Anspruch 5, wobei das Reinigungsmittel ein Reinigungsmittel zum Reinigen eines aus einem Metall, einem Harz, einem Kautschuk, einem Glas, einer Keramik, einer Naturfaser, einer synthetischen Faser oder einem zwei oder mehr davon umfassenden Verbundmaterial hergestellten Gegenstands ist.

10. Hydrohalogenolefinzusammensetzung nach Anspruch 5, wobei das Treibmittel mindestens eines, ausgewählt aus der Gruppe, bestehend aus einem Druckmittel und einem Zerstäubungsmittel, umfasst.

11. Hydrohalogenolefinzusammensetzung nach Anspruch 5, wobei das gasförmige Dielektrikum mindestens eines, ausgewählt aus der Gruppe, bestehend aus einem verflüssigten Gas und einem komprimierten Gas, umfasst.

## Revendications

1. Composition d'hydrohalogénooléfine comprenant une hydrohalogénooléfine comme composant principal, comprenant en outre du 1,1,2,3-tétrafluorobutane, dans laquelle une teneur en le 1,1,2,3-tétrafluorobutane va de 0,001 à 0,1 % en masse par rapport à une quantité totale de composés organiques.

2. Composition d'hydrohalogénooléfine selon la revendication 1, comprenant en outre du 2,4,4,4-tétrafluoro-1-butène,
dans laquelle une teneur totale en le 1,1,2,3-tétrafluorobutane et le 2,4,4,4-tétrafluoro-1-butène va de plus de 0,001 % en masse à 0,4 % en masse par rapport à une quantité totale de composés organiques.

3. Composition d'hydrohalogénooléfine selon la revendication 1 ou 2, dans laquelle le composant principal est une hydrochlorofluorooléfine.

4. Composition d'hydrohalogénooléfine selon la revendication 3, dans laquelle le composant principal est le 1-chloro-2,3,3,3-tétrafluoropropène.

5. Composition d'hydrohalogénooléfine selon l'une quelconque des revendications 1 à 4, qui est au moins un élément sélectionné parmi le groupe constitué d'un fluide actif, d'un agent moussant, d'un solvant diluant, d'un détergent, d'un propulseur, d'un agent extincteur, d'un diélectrique gazeux, d'un gaz de gravure et d'un gaz de couverture métallique fondu comme application.

6. Composition d'hydrohalogénooléfine selon la revendication 5, qui est sous une forme d'un aérosol.

7. Composition d'hydrohalogénooléfine selon la revendication 5, dans laquelle le fluide actif est un fluide actif pour cyclage thermique.

8. Composition d'hydrohalogénooléfine selon la revendication 5, dans laquelle le détergent est un détergent pour nettoyer au moins un élément sélectionné parmi le groupe constitué d'un carbone, d'une huile et d'une poussière adhérant par le biais de carbone ou d'huile.

9. Composition d'hydrohalogénooléfine selon la revendication 5, dans laquelle le détergent est un détergent pour nettoyer un article réalisé en un métal, une résine, un caoutchouc, un verre, une céramique, une fibre naturelle, une fibre synthétique ou un matériau composite comprenant deux éléments ou plus parmi ceux-ci.

10. Composition d'hydrohalogénooléfine selon la revendication 5, dans laquelle le propulseur comprend au moins un élément sélectionné parmi le groupe constitué d'un agent presseur et d'un agent d'atomisation.

11. Composition d'hydrohalogénooléfine selon la revendication 5, dans laquelle le diélectrique gazeux comprend au moins un élément sélectionné parmi le groupe constitué d'un gaz liquéfié et d'un gaz comprimé.
